# EUROPEAN PATENT APPLICATION

(11) **EP 0 524 621 A1**
(43) Date of publication of application: **27.01.1993**
(21) Application number: 92112571.2
(22) Date of filing: 23.07.1992
(51) Int. Cl.: C07C 17/26, C07C 19/08

(54) **Process for preparing 1,1,1,2,2-Pentafluoro-3, 3-Dichloropropane and 1,1,2,2,3-Pentafluoro-1,3-Dichloropropane**

(30) Priority: 23.07.1991 JP 182463/91
(71) Applicant: DAIKIN INDUSTRIES, LTD., Kita-ku, Osaka 530 (JP)
(72) Inventor: Aoyama, Hirokazu, c/o Yodogawa Works of Daikin, Settsu-shi, Osaka-fu (JP); Yasuhara, Takashi, c/o Yodogawa Works of Daikin, Settsu-shi, Osaka-fu (JP); Koyama, Satoshi, c/o Yodogawa Works Daikin, Settsu-shi, Osaka-fu (JP)
(74) Representative: Hansen, Bernd, Dr. Dipl.-Chem.

(57) **Abstract**

1,1,1,2,2-Pentafluoro-3,3-dichloropropane and 1,1,2,2,3-pentafluoro-1,3-dichloropropane are prepared by reacting tetrafluoroethylene a mixture of chloroform and difluorochloromethane in the presence of a catalyst selected from the group consisting of anhydrous titanium tetrachloride and a titanium chlorofluoride of the formula: TiClₓF_{y} in which x and y are independently a number of larger than 0 and smaller than 4 provided that a sum of x and y is 4, by which by-production of 2,2-dichloro-1,1,1,3,3-pentafluoropropane is suppressed.

## Description

### BACKGROUND OF THE INVENTION

### Field of the Invention

The present invention relates to a process for preparing 1,1,1,2,2-pentafluoro-3,3-dichloropropane (hereinafter referred to as "R-225ca") and 1,1,2,2,3-pentafluoro-1,3-dichloropropane (hereinafter referred to as "R-225cb"), which are substitute compounds for industrially important 1,1,2-trichloro-1,2,2-trifluoroethane and have less influence on the global environment.

### Description of the Related Art

Hitherto, as an industrial preparation process of R-225ca and R-225cb, there is is known a process comprising reacting tetrafluoroethylene (hereinafter referred to as "TFE") with dichlorofluoromethane (hereinafter referred to as "R-21") at a temperature of 15 to 100°C in the presence of anhydrous aluminum chloride as a catalyst (see U.S. Patent No. 2,462,402, J. Amer. Chem. Soc., 71, 979 and Collect. Czechoslov. Chem. Commun., 36, 1867).

But, Japanese Patent Kokai Publication No. 209824/1990 discloses that, when the same raw materials, namely TFE and R-21 is reacted at 0°C in the presence of the same catalyst (anhydrous aluminum chloride), 2,2-dichloro-1,1,1,3,3-pentafluropropane (hereinafter referred to as "R-225aa") is produced.

From this disclosure, it is easily expected that R-225aa would be by-produced when R-225ca and R-225cb are synthesized through the reaction of TFE and R-21 in the presence of anhydrous aluminum chloride. Actually, when a reaction mixture obtained from such reaction is analyzed, it is confirmed that a considerable amount of R-225aa is produced together with R-225ca and R-225cb.

When R-225ca and R-225cb are produced by the above process, the by-production of R-225aa is unavoidable so that yields and selectivities of R-225ca and R-225cb are not increased. Therefore, the above conventional process is not attractive for the industrial production of R-225ca and R-225cb.

In addition, when R-225aa is removed from a mixture of R-225ca, R-225cb and R-225aa to obtain pure R-225ca or a mixture of pure R-225ca and pure R-225cb, rectification, which is one of conventional purification and isolation methods, cannot effectively and economically remove R-225aa from the mixture of R-225ca, R-225cb and R-225aa, since a boiling point of R-225aa (51°C) is very close to that of R-225ca (51.1°C). Further, safety of R-225aa has not been confirmed and it cannot be said that R-225aa would cause no problem.

### SUMMARY OF THE INVENTION

An object of the present invention is to provide a process for preparing R-225ca and R-225cb with suppressing by-production of R-225aa.

According to the present invention, there is provided a process for preparing R-225ca and R-225cb comprising reacting TFE with a mixture of chloroform and difluorochloromethane (hereinafter referred to as "R-22") in the presence of a catalyst selected from the group consisting of anhydrous titanium tetrachloride and a titanium chlorofluoride of the formula:

TiClₓF_{y} (I)

wherein x and y are independently a number of larger than 0 and smaller than 4 provided that a sum of x and y is 4.

### DETAILED DESCRIPTION OF THE INVENTION

In the process of the present invention, when chloroform, R-22 and TFE are used as the raw materials, the catalyst according to the present invention is dissolved or suspended in chloroform and then R-22 and TFE are supplied at a predetermined molar ratio at predetermined flow rates.

As the reaction proceeds, a reaction mixture containing increasing amounts of R-225ca and R-225cb is separated from the catalyst to recover them. For the separation of the dissolved or suspended catalyst, a separation method in a liquid state such as filtration or centrifugation or a separation method in a gas state such as evaporation is employed.

Since the recovered reaction mixture contains no R-225aa, it is easily separated and isolated by a conventional method such as rectification to obtain pure R-225ca, pure R-225cb or a mixture thereof economically.

Using the catalyst according to the present invention, it is possible to remove chloroform from a mixture comprising R-225ca, R-225cb, R-225aa and chloroform which is obtained by other process using R-21 and TFE as the raw material in the presence of any other catalyst. To this end, a specific amount of the catalyst according to the present invention is added to such mixture and then R-22 and TFE are supplied at a predetermined molar ratio at predetermined flow rates at a specific temperature.

In the present invention, the reaction is preferably carried out by continuously supplying the raw materials and continuously recovering the products in view of economy. It is also possible to employ a semi-batchwise reaction in which certain amounts of the raw materials are charged in a reactor, reacted for a certain time and then the reaction products are recovered, or a batchwise reaction in which certain amounts of the raw materials are charged in a reactor and, after the completion of the reaction, the reaction products are recovered.

Alternatively, the reaction of the present invention can be carried out in a gas phase by filling the catalyst in a reaction tube and flowing the raw materials through the reaction tube at predetermined flow rates.

Though the reaction of the present invention may be carried out in the absence of a solvent, it can be carried out in the presence of a solvent which is inactive to the catalyst and in which R-22, chloroform and TFE are dissolved may be used.

For example, the solvent may be a chloroalkane such as chloroform which is one of the raw materials for the reaction of the present invention, or hydrochlorofluoroalkanes such as tetrafluorotrichloropropanes which are by-products in the reaction of the present invention. Further, a usually used solvent such as dichloromethane which is a kind of chloroalkane, or tetrachlorotetrafluoropropane or tetrachlorohexafluorobutane which are chlorofluoroalkanes may be used.

In view of the easiness of the reaction, chloroform is preferably used as the solvent. In view of the separation of R-225ca and R-225cb, R-225ca and R-225cb themselves are used as the solvents.

When chloroform, R-22 and TFE are used as the raw materials, a molar ratio of chloroform to R-22 is at least 1:1, preferably from 1:1 to 1:10, and a molar ratio of R-22 to TFE is at least 1:2, preferably from 1:2 to 1:10. A molar ratio of chloroform/R-22/TFE is for example 1:2:4.

The raw materials are premixed and then charged in the reactor, or they are separately charged in the reactor simultaneously. In some cases, a certain amount of chloroform is charged for a certain time period and then a mixture of R-22 and TFE is charged. The raw materials can be charged in a gas state or a liquid state.

The titanium chlorofluoride (I) used as the catalyst according to the present invention may be prepared by fluorinating anhydrous titanium tetrachloride with hydrogen fluoride or a chlorofluorocarbon, fluorohydrocarbon or chlorofluorohydrocarbon having 1 to 4 carbon atoms, preferably 1 or 2 carbon atoms (e.g. difluoromethane, trifluoromethane, tetrafluoroethane, pentafluoroethane, chlorotrifluoromethane, dichlorodifluoromethane, trifluorochloromethane, chloropentafluoroethane, dichlorotetrafluoroethane, trifluorotrichloroethane, difluorotetrachloroethane, chlorodifluoromethane, dichlorofluoromethane, trifluorodichloroethane, trifluorotrichloroethane, etc.).

In the above preparation step, hydrogen fluoride, or the chlorofluorocarbon, fluorohydrocarbon or chlorofluorohydrocarbon may be reacted alone, or a mixture of two or more of them may be reacted. Further, they may be used in combination with a chlorohydrocarbon.

The reaction temperature is from 0 to 120°C, preferably from 0 to 100°C. The above fluorination compound may be contacted with anhydrous titanium tetrachloride in the liquid state or the gas state.

For the preparation of the titanium chlorofluoride (I), there is known a process comprising reacting chlorine with titanium trifluoride to obtain titanium chlorotrifluoride (see Halides of the Transition Elements, Halides of the First Row Transition Metals, John Wiley and Sons, New York, 39 (1969); and J. Amer. Chem. Soc., 77, 201 (1955)).

An amount of the catalyst is not critical. The amount of the catalyst is usually from 0.01 to 30 % by weight, preferably from 0.1 to 20 % by weight based on the weight of the raw materials. When the solvent is used, the amount of the catalyst is usually from 0.01 to 50 % by weight, preferably from 0.1 to 30 % by weight based on the weight of the solvent.

A reaction temperature in the process of the present invention is usually from -30 to +150°C, preferably from -20 to 100°C. When the reaction temperature exceeds 150°C, amounts of the by-products increase while the selectivities of R-225ca and R-225cb decrease. When the reaction temperature is lower than -30°C, the reaction rate is extremely low and unpractical.

A reaction pressure is not critical either and a reduced pressure may be employed. Preferably, atmospheric pressure or higher is employed, since an apparatus becomes complicated when the reduced pressure is employed.

The raw materials, namely R-21, R-22, chloroform and TFE are industrially produced. As anhydrous titanium tetrachloride, a commercially available one is used as such.

### PREFERRED EMBODIMENTS OF THE INVENTION

### Example 1

In a SUS 316 made 200 ml autoclave equipped with a stirrer, anhydrous titanium tetrachloride (19 g) and trichlorofluoromethane (48 g) were charged and stirred at 170°C for 10 hours. After cooling down to room temperature, by-produced compounds and unreacted trichlorofluoromethane were removed under reduced pressure to obtain titanium chlorofluoride.

### Example 2

In a SUS 316 made 200 ml autoclave, titanium chlorofluoride (3 g) which was prepared in Example 1 and chloroform (30 g) were charged. After reducing the internal pressure of the flask, TFE was injected to 6 kg/cm²G at room temperature and then R-22 was injected till the pressure reached 8 kg/cm²G. Thereafter, the flask was heated to a temperature of 50 to 60°C while stirring. Since the internal pressure dropped as the reaction proceeded, a mixture of TFE and R-22 in a molar ratio of 2:1 was charged to keep the pressure at 8 kg/cm²G and the reaction was continued. When no pressure drop was observed, the reaction mixture in the flask was recovered. A weight of the reaction mixture was 98.5 g, and its composition was as follows:

| Compound | % by mole |
|---|---|
| R-225ca | 41.5 |
| R-225cb | 46.3 |
| Chloroform | 2.1 |
| R-224ca | 8.8 |
| Others | 1.3 |
| No R-225aa was detected. | |

### Example 3

In a SUS 316 made 100 ml autoclave, a mixture of R-225ca and R-225cb (molar ratio of 54:46) containing 3 % by mole of chloroform was charged and then titanium chlorofluoride which was prepared in the same manner as in Example 1 (2 g) was added. After reducing the internal pressure of the flask, TFE was injected to 6 kg/cm²G at room temperature and then R-22 was injected till the pressure reached 8 kg/cm²G. Thereafter, the flask was heated to a temperature of 50 to 60°C while stirring. Since the internal pressure dropped as the reaction proceeded, a mixture of TFE and R-22 in a molar ratio of 2:1 was charged to keep the pressure at 8 kg/cm²G and the reaction was continued. After 6 hours, the reaction mixture was recovered. A concentration of chloroform in the mixture of R-225ca and R-225cb decreased to 0.1 % by mole.

## Claims

1. A process for preparing 1,1,1,2,2-pentafluoro-3,3-dichloropropane and 1,1,2,2,3-pentafluoro-1,3-dichloropropane comprising reacting tetrafluoroethylene with a mixture of chloroform and difluorochloromethane in the presence of a catalyst selected from the group consisting of anhydrous titanium tetrachloride and a titanium chlorofluoride of the formula:
TiClₓF_{y} (I)
wherein x and y are independently a number of larger than 0 and smaller than 4 provided that a sum of x and y is 4.

2. The process according to claim 1, wherein the reaction is carried out in the presence of a solvent.

3. The process according to claim 2, wherein said solvent is at least one compound selected from the group consisting of 1,1,1,2,2-pentafluoro-3,3-dichloropropane and 1,1,2,2,3-pentafluoro-1,3-dichloropropane.

4. The process according to claim 2, wherein said solvent is a chloroalkane.

5. The process according to claim 4, wherein said chloroalkane is chloroform.

6. The process according to claim 1, wherein a molar ratio of chloroform to difluorochloromethane is at least 1:1, a molar ratio of difluorochloromethane to tetrafluoroethylene is at least 1:2.

7. The process according to claim 1, wherein a reaction temperature is from -30 to +150°C.
